# EUROPEAN PATENT APPLICATION

(11) **EP 1 917 973 A1**
(43) Date of publication of application: **07.05.2008**
(21) Application number: 07025192.1
(22) Date of filing: 02.06.2006
(51) Int. Cl.: A61K 38/55, A61K 9/20, A61K 31/395, A61K 31/33

(54) **Stable formulation comprising a moisture sensitive drug and manufacturing procedure thereof**

(62) Divisional of application: 06252877.3
(71) Applicant: Teva Pharmaceutical Industries Ltd, 49131 Petah Tiqva (IL)
(72) Inventor: Fox, Michael, Tel-Aviv 67291 (IL)
(74) Representative: Russell, Tim

(57) **Abstract**

The present invention provides stable pharmaceutical compositions comprising moisture sensitive drugs, in particular an angiotensin converting enzyme (ACE) inhibitor such as cilazapril, as the active ingredient, and a first pharmaceutical excipient, wherein the active pharmaceutical ingredient is wet granulated with a solution of the first pharmaceutical excipient, and methods for preparing such stable pharmaceutical compositions.

## Description

### FIELD OF THE INVENTION

The present invention relates to stable pharmaceutical compositions comprising moisture sensitive drugs, in particular an angiotensin converting enzyme (ACE) inhibitor, such as cilazapril, as the active ingredient and methods for preparing such stable pharmaceutical compositions.

### BACKGROUND OF THE INVENTION

Cilazapril is apparently an angiotensin converting enzyme ("ACE") inhibitor, which enzyme inhibits the formation of angiotensin II from angiotensin I by inhibiting the angiotensin converting enzyme. Chemically, cilazapril is reported to be (1S,9S)-9-[(S)-1-Ethoxycarbonyl-3-phenylpropylamino]-10-oxoperhydropyridazino[1,2-a][1,2]diazepine-1-carboxylic acid and is understood to be disclosed in U.S. Patent No. 4,512,924. Cilazapril has been prescribed in treating patients suffering from hypertension.

One of the requirements for an acceptable pharmaceutical composition is that it must be stable. A stable pharmaceutical composition does not exhibit substantial decomposition of the active pharmaceutical ingredient during the time between the manufacture of the composition and its use by a patient. Cilazapril and a number of other drugs suffer from instability problems because the active pharmaceutical ingredient rapidly degrades in the presence of water/moisture. Such active pharmaceutical ingredients (drugs) can therefore be characterized as moisture-sensitive drugs.

It is known that, tablet blends may be dry mixed, dry-granulated or wet-granulated before tableting. The choice of the processing procedure, dry mixing, dry granulation, wet granulation, or some other granulation process, depends on the properties of the drug and the chosen excipients. Generally, a dry manufacturing process is thought to be preferable for moisture-sensitive drugs.

To improve the stability of moisture sensitive drugs, water scavenger compounds may be incorporated into a tablet matrix. One such a water scavenger compound is the binder Copovidone (Plasdone S-630^{®}), which binder is specifically recommended for moisture sensitive drugs. However, with very little success attempts were made to formulate cilazapril tablets using this material in a dry granulation process. In such cilazapril tablets degradation of the active pharmaceutical ingredient was apparent.

Wet-granulation processes have not been considered appropriate for moisture sensitive drugs since the very nature of these processes can include the presence of water/moisture.

Surprisingly, we found that the best stability results can be achieved with a composition or formulation comprising the moisture sensitive drug and a first pharmaceutical excipient, such as a binder, e.g. copovidone, wherein the formulation/composition is prepared using a wet granulation process.

### SUMMARY OF THE INVENTION

The invention provides stable pharmaceutical compositions and methods of their preparation.

In one aspect, the present invention provides a stable pharmaceutical composition comprising;
a) a moisture sensitive active pharmaceutical ingredient; and
b) a first pharmaceutical excipient,
wherein the active pharmaceutical ingredient is wet granulated with a solution of the first pharmaceutical excipient. Preferably, the first pharmaceutical excipient is a binder.

In further aspect, the present invention provides a method of preparing a stable pharmaceutical composition of the present invention comprising the following steps of:
a) providing a moisture sensitive active pharmaceutical ingredient;
b) wet granulating the moisture sensitive active pharmaceutical ingredient with a solution comprising a first pharmaceutical excipient.

The present invention also provides a method of treating a patient suffering from a disease comprising administering to a patient in need thereof a therapeutically effective amount of a stable pharmaceutical composition comprising a moisture sensitive active pharmaceutical ingredient, preferably cilazapril, and a first pharmaceutical excipient, wherein the active pharmaceutical ingredient is wet granulated with a solution of the first pharmaceutical excipient.

### BRIEF DESCRIPTION OF THE FIGURES

- Figure 1.: Shows a comparison of the degradation at 55°C during stability test of various cilazapril tablets packed in aluminum cold-form blister, according to the invention, with a dry granulated tablet and a commercially available tablet. The increase of cilazaprilat, a major cilazapril degradation product, was determined.

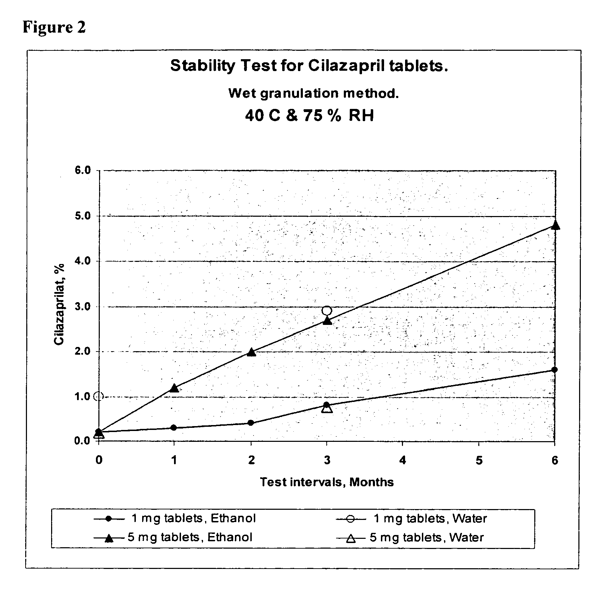

- Figure 2.: Shows the stability behavior of cilazapril tablets when comparing aqueous and ethanol based granulation processes.
- Figure 3.: Shows the stability behavior of cilazapril tablets when comparing a polyvinyl alcohol based tablet coating (Opadry II (85 Series) and a hydroxypropyl methylcellulose based tablet coating.

### DETAILED DESCRIPTION OF THE INVENTION

As used herein the term moisture sensitive active pharmaceutical ingredient refers to an active pharmaceutical ingredient which rapidly degrades in the presence of water/ moisture.

In one aspect, the present invention provides a stable pharmaceutical composition comprising a moisture sensitive active pharmaceutical ingredient, exemplified by cilazapril, and a first pharmaceutically acceptable excipient wherein the active pharmaceutical ingredient is wet granulated with a solution of the first pharmaceutical excipient.

The first pharmaceutically acceptable excipient is preferably a binder. More preferably, the binder comprises for example, one or more of, a cellulose derivative, a polyvinyl pyrrolidone (PVP) and its derivatives, a polyvinylacetate (PVA) or a polyvinyl alcohol. Examples of suitable cellulose derivatives as a binder in the present invention are hydroxypropylmethyl cellulose (HPMC) or hydroxypropyl cellulose (HPC). Most preferably, the binder is copovidone, exemplified by Plasdone^{®} S-630 (copovidone), which is a synthetic, 60:40, linear, random copolymer of N-vinyl-2-pyrrolidone and vinyl acetate, and which has a reduced hydrophilicity and a reduced polymer glass transition temperature (Tg) in comparison to a polyvinyl pyrrolidone (PVP) homopolymer.

Preferably, the stable pharmaceutical composition of the present invention comprises at least about 4% of the first pharmaceutical excipient by total weight of the composition. More preferably, the pharmaceutical composition comprises from about 4% to about 20%, most preferably from about 5% to about 10% of the first pharmaceutical excipient by total weight of the composition.

Suitable moisture sensitive active pharmaceutical ingredients are angiotensin converting enzyme (ACE) inhibitors. Most preferably, the moisture sensitive pharmaceutically active ingredient is cilazapril.

Preferably the amount of active pharmaceutical ingredient in the composition is about 0.1% to about 25%, more preferably about 0.5% to about 15%, of the total weight of the composition. A most preferred amount of active pharmaceutical ingredient in the composition is about 0.6% to about 2.7% of the total weight of the composition.

The pharmaceutical compositions of the present invention are stable. Preferably, the stable pharmaceutical composition contains not more than 3% (w/w of the initial amount of active pharmaceutical ingredient) of a degradation product of the active pharmaceutical ingredient after storage in a package with moisture sensitive barrier properties which are at least as efficient as aluminum-aluminum cold form blisters. Preferably, the concentration of degradation product in the stable pharmaceutical composition of the present invention after storage as described above is not more than 2%. More preferably, the concentration of degradation product in the stable pharmaceutical composition of the present invention after storage as described above is not more than 1%. Storage may comprise storage at a temperature of 55°C for 14 days and storage at a temperature of 40°C and 75% relative humidity for three months. The degradation product may be detected by HPLC analysis.

Preferably, the active pharmaceutical ingredient is cilazapril and the degradation product is cilazaprilat.

The stable pharmaceutical compositions of the present invention may further contain excipients such as tablet and capsule fillers and diluents (such as microcrystalline cellulose, lactose, starch and tri-basic calcium phosphate), disintegrants (such as starch, croscarmellose sodium, crospovidone and sodium starch glycolate), glidants (such as colloidal silicon dioxide and talc), and lubricants (such as magnesium stearate, sodium lauryl sulfate, stearic acid and sodium stearyl fumarate).

More particularly, suitable diluents and fillers for use in the pharmaceutical composition of the present invention include microcrystalline cellulose (e.g. Avicel^{®}), lactose, starch, pregelatinized starch, calcium carbonate, calcium sulfate, sugar, dextrates, dextrin, dextrose, dibasic calcium phosphate dihydrate, tribasic calcium phosphate, magnesium carbonate, magnesium oxide, maltodextrin, mannitol, powdered cellulose, sodium chloride, sorbitol and talc.

Solid pharmaceutical compositions of the present invention that are compacted into a dosage form, such as a tablet, may include the addition of a disintegrant to the composition. Disintegrants include croscarmellose sodium (e.g. Ac Di Sol^{®}, Primellose^{®}), crospovidone (e.g. Kollidon^{®}, Polyplasdone^{®}), microcrystalline cellulose, polacrilin potassium, powdered cellulose, pregelatinized starch, sodium starch glycolate (e.g. Explotab^{®}, Primoljel^{®}) and starch.

Glidants can be added to improve the flowability of a solid composition before compaction and to improve the accuracy of dosing especially during compaction and capsule filling. Excipients that may function as glidants include colloidal silicon dioxide, magnesium trisilicate, powdered cellulose, and talc.

A lubricant can be added to the composition to reduce adhesion and/or ease the release of the product from e.g. the dye. Lubricants include magnesium stearate, calcium stearate, glyceryl monostearate, glyceryl palmitostearate, hydrogenated castor oil, hydrogenated vegetable oil, mineral oil, polyethylene glycol, sodium lauryl sulfate, sodium stearyl fumarate, stearic acid, talc and zinc stearate.

Preferably, one or more further pharmaceutical excipients are wet granulated with the moisture sensitive active pharmaceutical ingredient and the first pharmaceutical excipient.

Other excipients that may be incorporated into the formulation include preservatives, surfactants, antioxidants, or any other excipient commonly used in the pharmaceutical industry.

In a preferred embodiment of the present invention, the stable pharmaceutical composition comprises cilazapril, copovidone, lactose monohydrate, sodium starch glycolate, talc and sodium stearyl fumarate.

The solid compositions of the present invention include powders, granulates, aggregates and compacted compositions. The dosages include dosages suitable for oral, buccal, and rectal administration. Although the most suitable administration in any given case will depend on the nature and severity of the condition being treated, the most preferred route of the present invention is oral. The dosages may be conveniently presented in unit dosage form and prepared by any of the methods well known in the pharmaceutical arts.

The pharmaceutical composition of the present invention may be prepared in any dosage form such as a compressed granulate in the form of a tablet for example. Also, uncompressed granulates and powder mixes that are obtained by the method of the present invention in the pre-compression steps can be simply provided in a dosage form of a capsule or sachet. Therefore, dosage forms of the pharmaceutical composition of the present invention include solid dosage forms like tablets, powders, capsules, sachets, etc. The dosage form of the present invention may also be a capsule containing the composition, preferably a powdered or granulated solid composition of the invention, within either a hard or soft shell. The shell may be made from gelatin and optionally contain a plasticizer such as glycerin and sorbitol, and an opacifying agent or colorant.

Once a moisture sensitive active pharmaceutical ingredient, preferably cilazapril, solid composition is prepared in accordance with the present invention, it is preferably formulated into pharmaceutical formulations such as conventional dosage forms, including tablets and capsules. Tablets are a preferred dosage form. In addition, the tablets may be coated with an optional cosmetic tablet coating. More preferably this cosmetic coating has "moisture barrier" properties. This moisture barrier property provides protection against environmental moisture for sensitive cores, enhances product stability, and improves shelf life.

Preferably, the cosmetic coating is a tablet coating based on polyvinyl alcohol. More preferably, the cosmetic coating comprises polyvinyl alcohol, talc and polyethylene glycol (PEG). Most preferably, the cosmetic coating further comprises an opacifier and/or a colorant, e.g. titanium dioxide and/or iron oxide.

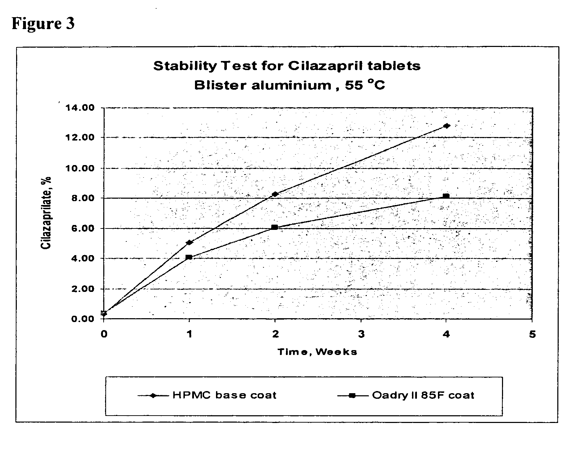

As shown in figure 3, a comparison is made between the stability of a tablet coated with Opadry®II 85F (a coating with moisture barrier properties/based on polyvinyl alcohol) and a tablet coated with an HPMC based coat The commercially available series of powder mixes for coating suspension sold as the Opadry^{®}II 85F series, available from Colorcon, which are based on polyvinyl alcohol, are examples of such a cosmetic coat. In addition to polyvinyl alcohol, this Opadry series of products comprise talc, PEG 3350, titanium dioxide and pigments.

Preferably, the tablets of the present invention comprise a cosmetic coat of about 2% to about 6% of the tablet weight, more preferably of about 2.5% to about 4.5% of the tablet weight, most preferably of about 3% to about 3.5% of the tablet weight.

In another embodiment, the present invention provides a method of preparing a stable pharmaceutical composition of the present invention, comprising the following steps of
a) providing a moisture sensitive active pharmaceutical ingredient;
b) wet granulating the moisture sensitive active pharmaceutical ingredient with a granulation solution comprising a first pharmaceutical excipient to form a granulate.

In preparing a pharmaceutical composition of the present invention a typical granulation process involves mixing the active ingredient and possible excipients in a mixer. The first pharmaceutical excipient is dissolved in the solvent used for granulating although a further portion of the first pharmaceutical excipient or another pharmaceutical excipient may be one of the excipients added in the dry mix state. The granulating solvent, solution or suspension is added to the dry powders in the mixer and mixed until the desired characteristics are achieved. This usually produces a granule that will be of suitable characteristics for producing tablets with adequate hardness, dissolution, content uniformity, and other physical characteristics. After the wet granulation step, the product is most often dried and more preferably then milled after drying to obtain a major percentage of the product within a desired size range. Preferably, the product after wet granulation is dried until the loss on drying (LOD) is not more than about 1.5%, more preferably not more than about 1.1%. Preferably, the product is milled or sized through an 1 mm screen, more preferably through a 0.8 mm screen.

Preferably, the stable pharmaceutical composition of the present invention is prepared by wet granulation with a suitable solvent/processing solvent. A suitable solvent/processing solvent is able to dissolve the selected first pharmaceutical excipient. Preferably, the solvent/processing solvent is capable of dissolving the first pharmaceutical excipient to reach a concentration of at least about 10% W/W. More preferably, the solvent/processing solvent is selected from the group consisting of ethanol, isopropyl alcohol, water, and combinations thereof.

Preferably, the stable formulation prepared by wet granulation comprises at least 4%, preferably about 4% to about 20%, more preferably about 5% to about 10%, of a first pharmaceutical excipient by weight of the formulation. Preferably, the first pharmaceutical excipient is applied as a solution in ethanol or water. A preferred solution of the first pharmaceutical excipient in ethanol or water comprises about 25% to about 55% (w/w) first pharmaceutical excipient, more preferably about 30% to about 50% (w/w).

Preferably, the first pharmaceutical excipient is a binder. More preferably, the binder is selected from the binders listed above. Most preferably, the binder is copovidone.

Surprisingly, it was determined that the choice of processing solvent used in wet granulation impacts the stability of the final product differently depending on the dose/amount of active pharmaceutical ingredient in the final product. Thus, for compositions comprising 1 mg of active pharmaceutical ingredient it was determined that a granulation processing solvent comprising predominantly ethanol, such as ethanol (95%), produces more stable pharmaceutical compositions than the same process wherein the processing solvent predominantly comprises water.

In contrast, with respect to pharmaceutical compositions comprising 5 mg of active pharmaceutical ingredient, it was determined that a granulation processing solvent comprising predominantly water (aqueous granulation) produces a more stable pharmaceutical composition than the same process wherein the processing solvent predominantly comprises ethanol (95%).

This effect may be characterized as related to the concentration of the active pharmaceutical ingredient in the dried granulate. Therefore, dried granulates comprising about 0.6% active pharmaceutical ingredient are preferentially prepared by wet granulation with an alcoholic granulation processing solvent, whereas dried granulates comprising about 2.7% active pharmaceutical ingredient are preferentially prepared by wet granulation with an aqueous granulation processing solvent. Granulates with intermediate concentrations of active pharmaceutical ingredient display an intermediate effect.

Therefore, final pharmaceutical compositions of the present invention comprising not more than about 1.7 % active pharmaceutical ingredient in the dried granulate are preferably prepared by wet granulation with an alcoholic granulation process solvent. Preferably, wet granulation with an alcoholic processing solvent is used for such compositions comprising not more than about 0.6% active pharmaceutical ingredient in the dried granulate. Pharmaceutical compositions comprising more than about 1.7% active pharmaceutical ingredient in the dried granulate are preferably prepared by wet granulation with water (an aqueous granulation) as the granulation processing solvent. Preferably, wet granulation with an aqueous processing solvent is used for such compositions comprising not less than about 2.7% active pharmaceutical ingredient in the dried granulate.

The method of the present invention may be adapted for preparing tablets and such a method further comprises the steps of:
c) mixing the granulate with one or more excipients forming a final blend;
d) pressing the final blend into a tablet; and
e) optionally coating the tablet with a cosmetic coating.

Preferably, the cosmetic coating has moisture barrier properties. Examples of such cosmetic coatings are tablet coatings based on polyvinyl alcohol as described above.

The optional cosmetic coating of the tablet preferably comprises preparing a suspension comprising about 10% to about 25%, preferably about 12% to about 15%, more preferably about 12% to about 13%, of a powder mixture for cosmetic coating, and applying the suspension on the tablet. The cosmetic coating suspension is preferably prepared such that the tablet comprises about 2% to about 6%, preferably 2.5% to about 4.5%, of a tablet cosmetic coat. The tablet cosmetic coat in the present invention preferably has "moisture barrier" properties. The commercially available series of powder mixes for coating suspension sold as the Opadry^{®}II 85F series, available from Colorcon, which are based on polyvinyl alcohol, are examples of such cosmetic coat.

Capsules comprising either a hard or soft shell and containing the composition of the present invention may be prepared. The shell may be made from gelatin and optionally contain a plasticizer such as glycerin and sorbitol, and an opacifying agent or colorant. A capsule tilling of the present invention may comprise the granulates that were described with reference to tableting, a final blend of a granulate composition of the present invention mixed with one or more excipients, however they are not subjected to a final tableting step. Further, such capsules may be prepared by any of the methods well known in the pharmaceutical arts.

The present invention also provides a method of treating a patient suffering from a disease comprising administering to a patient in need thereof a therapeutically effective amount of a pharmaceutical composition comprising a moisture sensitive active pharmaceutical ingredient, preferably Cilazapril, and at least one pharmaceutical excipient, wherein the active pharmaceutical ingredient is wet granulated with a solution of the at least one pharmaceutical excipient. Preferably, the disease is hypertension.

In a further embodiment, the present invention provides a stable pharmaceutical composition comprising a moisture sensitive pharmaceutical ingredient obtainable by a method as described above.

In a preferred embodiment, the invention provides a stable pharmaceutical composition obtainable by a process comprising:
i) mixing cilazapril, lactose, talc and sodium starch glycolate;
ii) adding a solution of copovidone to the mixture obtained from step i) to form a granulate;
iii) drying and then milling the granulate;
iv) combining the milled granulate with further sodium starch glycolate and mixing; and
v) adding sodium stearyl glycolate to the blend obtained from step iv) and mixing to obtain a final blend.

In a more preferred embodiment, the pharmaceutical composition is a 1 mg tablet and step ii) is performed using a granulation solution comprising ethanol.

In an alternative preferred embodiment, the pharmaceutical composition is a 5 mg tablet and step ii) is performed using an aqueous granulation solution.

In a yet further embodiment, the present invention provides a pharmaceutical composition as described above for use in therapy, preferably for treating hypertension.

The following examples are presented in order to further illustrate the invention. These examples should not be construed in any manner to limit the invention.

### EXAMPLES

### Example 1.

**1 mg tablets, dry granulation, *~5% (w/w) binder** (Comparative example)

### (* % of the binder calculated per tablet core)

In a polyethylene bag 8.4 g Cilazapril Monohydrate, 1360g Lactose Monohydrate, 64g Talc Extra Fine and 80g Copovidone were mixed. The blend was screened through a 0.71 mm screen, transferred to a twin-shelled (Y-cone) dry blender and mixed for 25 minutes. To this mixture 16g of screened Sodium Stearyl Fumarate was added and all the materials were mixed in a Y-cone blender for 5 minutes.

The blend was pressed into slugs on a rotary tablet press and the slugs were milled to a granulate in an oscillating granulator through 0.8 mm screen. The obtained granulate was combined with 64g screened Sodium Starch Glycolate (type A) and mixed in a Y-cone blender for 10 minutes. To the granulate mixture 8g screened Sodium Stearyl Fumarate was added and all materials were mixed in a Y-cone blender for 5 minutes,

Tablets were pressed in a rotary tablet press. Subsequently, a part of the tablets cores were coated with:
a) Opadry^{®} II 85F22055 (Yellow), which comprises polyvinyl alcohol, talc, PEG 3350, titanium dioxide and iron oxide, as a 13% aqueous suspension, using an Glatt film coater, to obtain approximately a 2.7 % w/w coating. The tablets were then packaged in aluminum blisters covered with aluminum foil, (cold - form Aluminum blisters).
   Another part of the tablets cores were coated with:
b) Opadry^{®} 02G222555 (Yellow), which comprises Hydroxypropyl Methylcellulose (HPMC), talc, PEG, titanium dioxide and iron oxide, as a 11% aqueous suspension, using an Glatt film coater, to obtain approximately a 2.2 % w/w coating. The tablets were then packaged in aluminum blisters covered with aluminum foil, (cold - form Aluminum blisters)

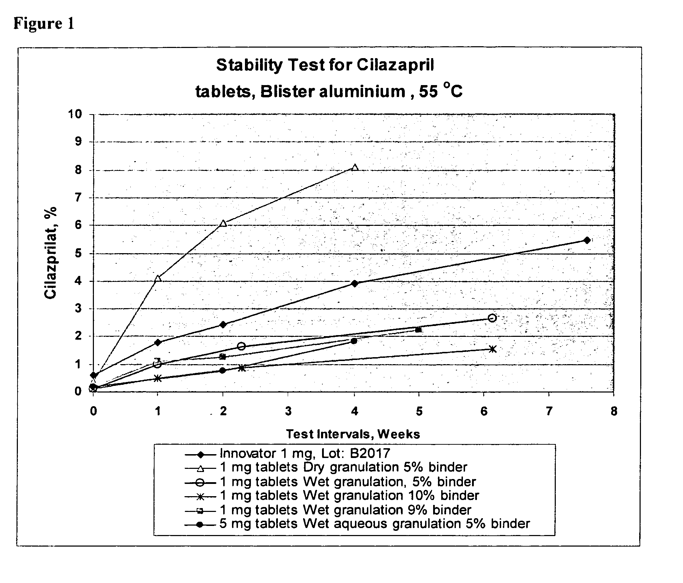

Packaged tablets were either stored at 55°C or at 40°C and 75% relative humidity (RH). The presence of the main degradation product, Cilazaprilat, was determined using the HPLC method. In figure 1 the presence of this main degradation product of Cilazapril after such storage is shown. Also, figure 3 compares the degradation of Cilazapril tablets, as a function of the presence of this main degradation product after storage, of tablets coated with a cosmetic coating of Opadry®II 85 F22055 with tablets having a cosmetic coat of Opadry® 02G222555 (HPMC based).

### Example 2.

### 1 mg tablets, wet granulation, ~5% (w/w) binder

2.1 g Cilazapril Monohydrate, 333.9 g Lactose Monohydrate, 16g Talc Extra Fine and 16g Sodium Starch Glycolate (type A) were mixed for 1 minute in a high shear mixer. 70 g of a 28.6% (w/w) solution of Copovidone (binder) in Alcohol (95%) was added and mixed in the high shear mixer for 2.5 minutes. 10 g of Alcohol (95%) was added and mixed for 1 minute. The obtained granulate was dried using a fluid bed dryer until the Loss On Dry (LOD) of the dried granulate (as measured by Mettler FIR73 at 80°C, level 5) was not more than (NMT) 1.1%. The granulate was milled or "sized" in an oscillating granulator through 0.8 mm screen.

The milled granulate was combined with 8g Sodium Starch Glycolate -type A (disintegrant) and mixed in a Y-cone blender for 10 minutes. 4g screened Sodium Stearyl Fumarate (lubricant) was added to the blend and mixed for 5 minutes to obtain a final blend.

Tablets were pressed from the final blend in a rotary tablet press. The tablets were coated with a commercially available tablet coating powder blend Opadry®II 85F22055 (Yellow) as a 12% aqueous suspension, using a Glatt film coater, to obtain approximately a 3% w/w coating.

The tablets were packaged in aluminum blister covered with aluminum foil. Packaged tablets were stored at 55°C. The presence of the main degradation product, Cilazaprilat, was determined using HPLC method.

### Example 3.

### 1 mg tablets, wet granulation, ~9% (w/w) binder

20.1 g Cilazapril Monohydrate, 3099.1 g Lactose Monohydrate, 160g Talc Extra Fine and 160g Sodium Starch Glycolate (type A) were mixed for 2 minutes in a high shear mixer;. 790 g of a 45.57% (w/w) solution of Copovidone in Alcohol (95%) was added and mixed in a high shear mixer for 5 minutes. The obtained granulate was dried using a fluid bed dryer until the Loss On Dry (LOD) of the dried granulate was not more than (NMT) 1.1 % as tested at 80°C. The dried granulate was milled in a hammer mill through a 0.84 mm screen.

The milled granulate was combined with 160g screened Sodium Starch Glycolate (type A) and mixed in a Y-cone blender for 10 minutes. 40g screened Sodium Stearyl Fumarate was added to the blend and mixed in a Y-cone blender for 5 minutes to obtain a final blend.

Tablets were pressed from the final blend in a rotary tablet press. The tablets were coated with Opadry® II 85F22055 Yellow as a 13% aqueous suspension, using an O'HARA film coater, to obtain approximately a 3.5% w/w coating.

The tablets were packaged in aluminum blister covered with aluminum foil. Packaged tablets were stored either at 55°C or at 40°C and 75% RH. The presence of the main degradation product, Cilazaprilat, was determined using the HPLC method.

### Example 4.

### 1 mg tablets, wet granulation, ~10% (w/w) binder

2.1 g Cilazapril Monohydrate, 305.9 g Lactose Monohydrate, 16g Talc Extra Fine and 16g Sodium Starch Glycolate (type A) were mixed for 1 minute in a high shear mixer. 105 g of a 38.1% (w/w) solution of Copovidone in Alcohol (95%) was added and mixed in a high shear mixer for 1 minute. The granulate obtained was dried using a fluid bed dryer until the Loss On Dry (LOD) of the dried granulate was not more than (NMT) 1.1 % as tested at 80°C. The dried granulate was milled in an oscillating granulator through a 0.8 mm screen.

The milled granulate was combined with 16g Sodium Starch Glycolate (type A) and mixed in a Y-cone blender for 10 minutes. 4g screened Sodium Stearyl Fumarate was added to the blend and mixed in a Y-cone blender for 5 minutes to obtain a final blend.

Tablets were pressed on a rotary tablet press. The tablets were coated with Opadry^{®} II 85F22055 Yellow as a 12% aqueous suspension, using a Glatt film coater, to obtain approximately a 3% w/w coating.

The tablets were packaged in aluminum blister covered with aluminum foil. Packaged tablets were stored at 55°C. The presence of the main degradation product, Cilazaprilat, was determined using an HPLC method.

### Example 5. (R- 02636)

### 5 mg tablets, wet aqueous granulation, ~5% (w/w) binder

The following components were mixed for 1 minute in a high shear mixer; 10.4 g Cilazapril Monohydrate, 318g Lactose Monohydrate, 16g Talc Extra Fine and 16 g Sodium Starch Glycolate (type A). 50 g of a 40% (w/w) aqueous solution of Copovidone was added and mixed in the high shear mixer for 5 minutes. The granulate achieved was dried using a fluid bed dryer until the Loss On Dry (LOD) of the dried granulate was not more than (NMT) 1.1 % as tested at 80 o C. the dried granulate was milled in an oscillating granulator through 0.8 mm screen.

The milled granulate was combined with 16g of screened Sodium Starch Glycolate (type A) and mixed in a Y-cone blender for 10 minutes. To the resultant blend 4g of screened Sodium Stearyl Fumarate was added and mixed in a Y-cone blender for 5 minutes to achieve a final blend.

The tablets were pressed in a rotary tablet press. The tablets were packaged in aluminum blisters covered with aluminum foil. Packaged tablets were stored at either 55°C or at 40°C and 75% RH. The presence of the main degradation product, Cilazaprilat, was determined using an HPLC method.

### Example 6.

### Stability comparisons of various Cilazapril pharmaceutical compositions.

The stability of pharmaceutical compositions according to the present invention were compared with the stability of a dry granulated comparative example of a Cilazapril tablet and with a commercialized product. The samples of the commercialized product were Vascace^{®} 1 mg tablets, produced by F. Hoffmann-La Roche Ltd, Basel, Switzerland. Table 1 shows the formulations of these pharmaceutical compositions with the exception of the commercialized product which was obtained as a finished product.

**Table 1. Comparison of formulations and manufacturing methods.**

| | ***Example 1** | **Example 2** | **Example 3** | **Example 4** | **Example 5** |
|---|---|---|---|---|---|
| | **1 mg tablets, Dry granulation, 5% binder** | **1 mg tablets, Wet granulation, 5% binder** | **1 mg tablets, Wet granulation, 9% binder** | **1 mg tablets, Wet granulation, 10% binder** | **5 mg tablets Wet granulation, 5% binder** |
| **Ingredient** | **Content, % of the total tablet weight** | | | | |
| Cilazapril Monohydrate | 4.51 | 0.51 | 0.50 | 0.51 | 2.61 |
| Lactose Monohydrate | 82.50 | 81.05 | 74.86 | 74.25 | 79.39 |
| Talc Extra Fine | 3.88 | 3.88 | 3.86 | 3.88 | 4.00 |
| Sodium Starch Glycolate | 3.88 | 5.83 | 7.73 | 7.77 | 8.00 |
| Copovidone | 4.85 | 4.85 | 8.70 | 9.71 | 5.00 |
| Sodium Stearyl Fumarate | 1.46 | 0.97 | 0.97 | 0.97 | 1.00 |
| Opadry II 85F22055 Yellow | 2.91 | 2.91 | 3.38 | 2.91 | N/A |
| Granulation process solvent | N/A | Alcohol | Alcohol | Alcohol | Water |

| | | | | | |
|---|---|---|---|---|---|
| * comparative example | | | | | |

Stability was measured by determining the presence of the major Cilazapril degradation product Cilazaprilat in the pharmaceutical composition after storage. An HPLC test method was applied to determine the quantity of the degradation products of Cilazapril. The mobile phase was a mixture of triethylamine buffer, tetrahydrofuran and acetonitrile. The detector was a UV spectrophotometer set at 214 nm.

Figure 1 shows stability test results comparing degradation after storage at 55°C for 14 days of various Cilazapril tablets, prepared according to the invention by wet granulation process, with the (control) tablets prepared by dry granulatio and commercially available tablets. All tested tablets were packed in aluminum blisters. The commercially available product is also packed in aluminium blister. The presence of increasing levels of Cilazaprilat over time was determined. Further, test results for degradation of some of these formulations under standard stress conditions are shown in Table 2.

**Table 2. Degradation under standard "stress" conditions (40°C and 75% RH), of Cilazapril displayed as function of different formulations and manufacturing methods.**

| **Description** | **1 mg tablets, *Dry granulation, 5% binder** | **1 mg tablets, Wet granulation** (ethanol) 9 % binder** | **5 mg tablets, Wet granulation*** (aqueous) 5 % binder** | **1 mg tablets, Vascace^{®}, Lot: B2017** |
|---|---|---|---|---|
| **Major degradation product, Cilazaprilat, Time "Zero". % per labeled claim of Cilazapril** | 0.4 | 0.2 | 4.2 | 0.6 |
| **Major degradation product, Cilazaprilat, storage period 3 months. % per labeled claim of Cilazapril** | 8.7 | 0.8 | 0.8 | 2.4 |

| | | | | |
|---|---|---|---|---|
| * Comparative example (example 1) ** Example 3 *** Example 5 | | | | |

### Example 7.

**1 mg tablets, wet granulation, ~9% (w/w) binder** (% of the binder calculated per tablet core), (batch No: K-33603, as shown in table 3).

In a high shear mixer the following components were mixed for 2 minutes; 20.9 g Cilazapril Monohydrate, 3099.1 g Lactose Monohydrate, 160 g Talc Extra Fine and 160g Sodium Starch Glycolate (type A). 770 g of a 46.8% (w/w) solution of Copovidone in Alcohol (95%) was added and mixed in a high shear mixer for 5 minutes. The granulate obtained was dried using a fluid bed dryer. The LOD of the dried granulate was not more than (NMT) 1.1% as tested by Mettler HR73 at 80°C, level 5. The dried granulate was milled in a hammer mill through a 0.84 mm screen.

The milled granulate was combined with 160g screened Sodium Starch Glycolate (type A) and mixed in a Y-cone blender for 15 minutes. 40g screened Sodium Stearyl Fumarate was added to the blend and the materials mixed in a Y-cone blender for 5 minutes to obtain a final blend.

Tablets were pressed in a rotary tablet press. The tablets were coated with Opadry^{®} II 85F22055 Yellow as a 13% aqueous suspension, using an O'HARA film coater, to obtain approximately a 3.5% w/w coating.

The tablets were packaged in aluminum blisters covered with aluminum foil. Packaged tablets were stored at 40°C and 75% RH. The presence of the main degradation product, Cilazaprilat, was determined using the HPLC method described above.

### Example 8.

### 2.5 mg tablets, wet granulation, *~9% (w/w) binder

*(% of the binder calculated per tablet core), (batch K-33604, as is shown in table 3).

The following components were mixed for 2 minutes in a high shear mixer; 52.2 g Cilazapril Monohydrate, 3068g Lactose Monohydrate, 160.00 g Talc Extra Fine and 160.00 g Sodium Starch Glycolate (type A). Added 770 g of a 46.8% (w/w) solution of Copovidone in Alcohol (95%) and mixed in a high shear mixer for 5 minutes. The obtained granulate was dried using a fluid bed dryer (LOD of the dried granulate was not more than (NMT) 1.1% as tested by a Mettler HR73 at 80°C, and milled in a hammer mill through 0.84 mm screen.

The milled granulate was combined with 160.00 g screened Sodium Starch Glycolate (type A) and mixed in a Y-cone blender for 15 minutes. To the mix 40.00 g screened Sodium Stearyl Fumarate was added and the materials mixed in a Y-cone blender for 5 minutes.

The tablets were pressed in a rotary tablet press. The tablets were coated with Opadry^{®} II 85F24033 Pink as a 13% aqueous suspension, using O'HARA film coater, to obtain approximately a 3.5% w/w coating.

The tablets were packaged in aluminum blisters covered with aluminum foil. Packaged tablets were stored at 40°C and 75% RH. The presence of the main degradation product, Cilazaprilat, was determined using the HPLC method as described.

### Example 9.

### 5 mg tablets, wet granulation. ~9% (w/w) binder

(% of the binder calculated per tablet core), (batch K-33749, as is shown in table 3),

The following components were mixed for 2 minutes in a high shear mixer; 522 g Cilazapril Monohydrate, 30678 g Lactose Monohydrate, 1600g Talc Extra Fine and 1600g Sodium Starch Glycolate (type A). 6600g of a 54.55% (w/w) solution of Copovidone in Alcohol (95%) was added and mixed in the high shear mixer for 3.5 minutes. The granulate obtained was dried using a fluidized bed dryer until the LOD of the dried granulate was not more than (NMT) 1.1% as tested by a Mettler HR73 at 80°C, level 5. The dried granulate was milled in a hammer mill through a 0.84 mm screen.

The milled granulate was combined with 1600 g screened Sodium Starch Glycolate (type A) and mixed in a Y-cone blender for 15 minutes. 400 g screened Sodium Stearyl Fumarate was added to the blend and the materials mixed in a Y-cone blender for 5 minutes to obtain a final blend.

The tablets were pressed from the final blend in a rotary tablet press, The tablets were coated with Opadry^{®} II 85F25401 Red as a 13% aqueous suspension, using an O'HARA film coater, to obtain approximately a 3.5% w/w coating.

The tablets were packaged in aluminum blister covered with aluminum foil. Packaged tablets were stored at 40°C and 75% RH. The presence of the main degradation product, Cilazaprilat, was determined using the HPLC method as described.

**Table 3. Compositions of Cilazapril tablets, formulated by wet granulation process. Ethanol (95%) was used as a process solvent.**

| | **Example 7** | **Example 8** | **Example 9** |
|---|---|---|---|
| | **1 mg tablets, K-33603** | **2.5 mg tablets, K-33604** | **5 mg tablets, K-33749** |
| **Ingredient** | **% Content, of the total tablet weight** | | |
| Cilazapril Monohydrate | 0.50 | 1.26 | 1.26 |
| Lactose Monohydrate | 74.86 | 74.10 | 74.10 |
| Talc Extra Fine | 3.86 | 3.86 | 3.86 |
| Sodium Starch Glycolate | 7.73 | 7.73 | 7.73 |
| Copovidone | 8.70 | 8.70 | 8.70 |
| Sodium Stearyl Fumarate | 0.97 | 0.97 | 0.97 |
| Opadry II 85F22055 Yellow | 3.38 | | |
| Opadry 1185F24033 Pink | | 3.38 | |
| Opadry II 85F25401 Red | | | 3.38 |
| *Ethanol 95% (process solvent) | 10.3 | 10.3 | 9.9 |

| | | | |
|---|---|---|---|
| *Removed during the drying process | | | |

### Example 10.

**Batch R-02474 - 1 mg tablets, wet aqueous granulation, ~7.5% (w/w) binder,** (as shown in table 4).

In a high shear mixer were mixed for 1 minute; 2.09 g Cilazapril Monohydrate, 315.91 g Lactose Monohydrate, 16.00 g Talc Extra Fine and 16.00 g Sodium Starch Glycolate (type A). 65 g of a 46.2% (w/w) aqueous solution of Copovidone was added and mixed in a high shear mixer for 4 minutes. The obtained granulate was dried using a fluid bed dryer (LOD of the dried granulate was not more than (NMT) 1.1% as tested by a Mettler HR73 at 80°C, level 5) and milled in an oscillating granulator through 0.8 mm screen.

The milled granulate (359.34 g) was combined with 15.13 g screened Sodium Starch Glycolate (type A) and mixed in a Y-cone blender for 15 minutes. To the mix 3.78 g screened Sodium Stearyl Fumarate was added and the materials were mixed in a Y-cone blender for 5 minutes.

The tablets were pressed in a single punch tablet press. The tablets were packaged in aluminum blister covered with aluminum foil. Packaged tablets were stored either at 55°C or at 40°C and 75% RH. The presence of the main degradation product, Cilazaprilat, was determined using the HPLC method.

**Table 4. Compositions of Cilazapril tablets, formulated by wet granulation process. Water was used as a process solvent.**

| | **1 mg tablets, R-02474** | **5 mg tablets, R-02636** |
|---|---|---|
| **Ingredient** | **% Content, of the total tablet weight** | |
| Cilazapril Monohydrate | 0.52 | 2.61 |
| Lactose Monohydrate | 78.98 | 79.39 |
| Talc Extra Fine | 4.00 | 4.00 |
| Sodium Starch Glycolate | 8.00 | 8.00 |
| Copovidone | 7.50 | 5.00 |
| Sodium Stearyl Fumarate | 1.00 | 1.00 |
| *Water (process solvent) | 8.75 | 7.50 |

| | | |
|---|---|---|
| *Removed during the drying process | | |

### Example 11.

### Stability comparisons of various Cilazapril pharmaceutical compositions.

The stability of pharmaceutical compositions according to the present invention prepared with either ethanol (95%) or water as the processing solvent were compared. In addition, the stability of commercially available products was determined under the same testing conditions. The samples of the commercially available product were Vascace^{®} tablets, produced by F. Hoffmann-La Roche Ltd, Basel, Switzerland. As is shown in table 5 comparable formulations of Cilazapril processed predominantly using ethanol, which only differ with respect to the content of Cilazapril in mg/tablet have very different degradation profiles, such that the 1mg tablets are the most stable and the 5mg the least stable.

**Table 5. Monitoring results of Cilazapril tablets, placed under standard stress conditions (40°C & 75% RH), in comparison to a commercially available product. Packaging - aluminum blister.**

| | **Test samples** | | | **Vascace^{™}** | | |
|---|---|---|---|---|---|---|
| **Batch** | **K-33603** | **K-33604** | **K-33749** | **#B2022** | **#B2141** | **#B2117** |
| **Strength** | **1 mg** | **2.5 mg** | **5 mg** | **1 mg** | **2.5 mg** | **5 mg** |

| **Major degradation product, Cilazaprilat** | | | | | | |
|---|---|---|---|---|---|---|
| **Test Interval** | **% per labeled claim of Cilazapril** | | | | | |
| **Time "0"** | 0.2 | 0.2 | 0.2 | 0.9 | 0.5 | 0.4 |
| **1 Month** | 0.3 | 0.8 | 1.2 | 2.0 | 1.4 | 0.8 |
| **2 Months** | 0.4 | 1.1 | 2.0 | 2.5 | 1.7 | 1.0 |
| **3 Months** | 0.8 | 2.2 | 2.7 | 3.5 | 2.3 | 1.3 |
| **6 Months** | 1.6 | 4.2 | 4.8 | 5.1 | 3.4 | 1.8 |

In contrast, table 6 shows that comparable formulations of Cilazapril processed predominantly using water as processing solvent which only differ with respect to the content of Cilazapril in mg/tablet have the opposite degradation characteristics, such that the 5mg tablets are the most stable and the 1mg the least stable.

**Table 6. Monitoring results of Cilazapril tablets, placed under standard stress conditions (40°C & 75% RH). Packaging - blister aluminium.**

| **Batch** | **R-02474** | **R-02636** |
|---|---|---|
| **Strength** | **1 mg** | **5 mg** |
| **Test Interval** | **Cilazaprilat, % per labeled claim of Cilazapril** | |
| **Time "0"** | 1.0 | 0.2 |
| **3 Months** | 2.9 | 0.8 |

## Claims

1. A stable pharmaceutical composition comprising:
a) a moisture sensitive active pharmaceutical ingredient; and
b) a first pharmaceutical excipient,
wherein the active pharmaceutical ingredient is wet granulated with a solution of the first pharmaceutical excipient.

2. The pharmaceutical composition according to claim 1, wherein the first pharmaceutical excipient is a binder.

3. The pharmaceutical composition according to claim 2, wherein the binder is selected from the group consisting of cellulose derivatives, polyvinyl pyrrolidones (PVP) and their derivatives, polyvinylacetates (PVA), polyvinyl alcohols and mixtures thereof.

4. The pharmaceutical composition according to claim 3, wherein the binder is copovidone.

5. The pharmaceutical composition according to claim 4, wherein copovidone is Plasdone S-630.

6. The pharmaceutical composition according to any one of the preceding claims, wherein the first pharmaceutical excipient is present in amount at least about 4% by total weight of the composition.

7. The pharmaceutical composition according to claim 6, wherein the first pharmaceutical excipient comprises about 4% to about 20% by total weight of the composition.

8. The pharmaceutical composition according to claim 7, wherein the first pharmaceutical excipient is present in an amount from about 5% to about 10% by total weight of the composition.

9. The pharmaceutical composition according to any one of the preceding claims, wherein the moisture sensitive pharmaceutical ingredient is present in amount from 0.1% to about 25.0% by total weight of the composition.

10. The pharmaceutical composition according to any one of the preceding claims, wherein the moisture sensitive active pharmaceutical ingredient is cilazapril.

11. The pharmaceutical composition according to any one of the preceding claims, wherein the pharmaceutical composition comprises further pharmaceutical excipients.

12. The pharmaceutical composition according to claim 11, wherein one or more further pharmaceutical excipients are wet granulated with the moisture sensitive active pharmaceutical ingredient and the first pharmaceutical excipient.

13. The pharmaceutical composition according to any one of the preceding claims, wherein the level of degradation product(s) deriving from the active pharmaceutical ingredient is not more than 3% by weight of the initial amount of active pharmaceutical ingredient, after storage in a package, wherein the package has moisture barrier properties at least as efficient as aluminum-aluminum cold form blisters.

14. The pharmaceutical composition according to claim 13, wherein the storage is at a temperature of 55°C for 14 days.

15. The pharmaceutical composition according to claim 13, wherein the storage is at a temperature of 40°C and relative humidity of 75% for 3 months.

16. The pharmaceutical composition according to any one of claims 13 to 15, wherein the level of degradation product(s) is not more than about 2% by weight of the initial amount of active pharmaceutical ingredient.

17. The pharmaceutical composition according to claim 16, wherein the level of degradation product(s) is not more than about 1% by weight of the initial amount of active pharmaceutical ingredient.

18. The pharmaceutical composition according to anyone of claims 13 to 17, wherein the active pharmaceutical ingredient is cilazapril and the degradation product is cilazaprilat.

19. The pharmaceutical composition according to any one of the preceding claims, wherein the pharmaceutical composition is in a solid dosage form selected from the group consisting of tablets, powders, capsules, sachets, troches and losenges.

20. The pharmaceutical composition according to claim 19, wherein the solid dosage form is a tablet.

21. The pharmaceutical composition according to claim 20, wherein the composition comprises cilazapril, copovidone, lactose monohydrate, sodium starch glycolate, talc and sodium stearyl fumarate.

22. The pharmaceutical composition according to claim 20 or claim 21, wherein the tablet comprises a cosmetic tablet coating.

23. The pharmaceutical composition according to claim 22, wherein the tablet comprises about 2% to about 6% by weight of a cosmetic tablet coating.

24. The pharmaceutical composition according to claim 23, wherein the tablet comprises about 2.5% to about 4.5% by weight of a cosmetic tablet coating.

25. The pharmaceutical composition according to any one of claims 21 to 24, wherein the cosmetic tablet coating has moisture barrier properties.

26. The pharmaceutical composition according to claim 25 wherein the cosmetic tablet coating is based on polyvinyl alcohol.

27. The pharmaceutical composition according to claim 26 wherein the cosmetic tablet coating comprises polyvinyl alcohol, talc, polyethylene glycol, and an opacifier and/or a colorant.

28. The pharmaceutical composition according to claim 27 wherein the cosmetic tablet coat is prepared using powder mixtures for coating suspensions of the Opadry^{®} II 85F series.

29. A method of preparing a pharmaceutical composition as defined in any one of the preceding claims comprising the following steps of:
a) providing a moisture sensitive active pharmaceutical ingredient;
b) wet granulating the moisture sensitive active pharmaceutical ingredient with a solution comprising a first pharmaceutical excipient and a process solvent to form a granulate.

30. The method according to claim 29, wherein the moisture sensitive active pharmaceutical ingredient is first mixed with the first pharmaceutical excipient before carrying out step b).

31. The method of claim 29 or claim 30, wherein the first pharmaceutical excipient is a binder.

32. The method according to any one of claims 29 to 31, wherein the process solvent is selected from the group consisting of solvents capable of dissolving the first pharmaceutical excipient to reach a concentration of at least 10% W/W.

33. The method according to claim 32, wherein the process solvent is selected from the group consisting of water, ethanol, isopropyl alcohol, and combinations thereof.

34. The method according to claim 33, wherein the process solvent is a concentrated ethanol solution and wherein the concentration of the moisture sensitive active pharmaceutical ingredient in the pharmaceutical composition is not more than about 1.7%.

35. The method according to claim 34, wherein the concentration of moisture sensitive active pharmaceutical ingredient is not more than about 0.6% in the pharmaceutical composition.

36. The method according to claim 33, wherein the process solvent is water and wherein the concentration of the moisture sensitive active pharmaceutical ingredient is more than about 1.7% in the pharmaceutical composition.

37. The method according to claim 35, wherein the concentration of moisture sensitive active pharmaceutical ingredient is not less than about 2.7% in the pharmaceutical composition.

38. The method according to any one of claims 29 to 37, further comprising the steps of
c) mixing the granulate with one or more further excipients forming a final blend; and
d) pressing the final blend into a tablet.

39. The method according to claim 38, further comprising the steps of
e) coating the tablet with a cosmetic tablet coating.

40. The method of claim 39, wherein the cosmetic tablet coating has moisture barrier properties.

41. The method according to claim 40, wherein the cosmetic coating comprises a powder mixture for coating suspensions of the Opadry II 85F series.

42. The method according to claim 41, further comprising a step of providing the powder mixture for coating suspensions of the Opadry^{®} II 85F series in a solution or suspension comprising about 10% to about 25% of the cosmetic tablet coating powder mixture.

43. The method according to claim 42, wherein the powder mixture for coating suspensions of the Opadry^{®} II 85F series is provided in a solution or suspension comprising about 12% to about 13% of the cosmetic tablet coating powder mixture.

44. A pharmaceutical composition comprising a moisture sensitive pharmaceutical active ingredient obtainable by a process as defined in any one of claims 29 to 43.

45. A pharmaceutical composition according to claim 44 obtainable by a process comprising:
i) mixing cilazapril, lactose, talc and sodium starch glycolate;
ii) adding a solution of copovidone to the mixture obtained from step i) to form a granulate;
iii) drying and then milling the granulate;
iv) combining the milled granulate with further sodium starch glycolate and mixing; and
v) adding sodium stearyl glycolate to the blend obtained from step iv) and mixing to obtain a final blend.

46. A pharmaceutical composition as defined in any one of claims 1 to 28 for use in therapy.

47. A pharmaceutical composition as defined in any one of claims 1 to 28 for use in treating hypertension.

48. Use of a moisture sensitive active pharmaceutical ingredient in the manufacture of a pharmaceutical composition for treating hypertension, wherein the moisture sensitive active pharmaceutical ingredient is wet granulated with a solution of the first pharmaceutical excipient to form a granulate.
